(19) Europäisches Patentamt
European Patent Office
Office européen des brevets

(11) Veröffentlichungsnummer: **0 000 334**
**B1**

(12) **EUROPÄISCHE PATENTSCHRIFT**

(45) Veröffentlichungstag der Patentschrift: **12.08.81**

(21) Anmeldenummer: **78100180.5**

(22) Anmeldetag: **16.06.78**

(51) Int. Cl.³: **C 07 D 239/48,**
A 61 K 31/505,
C 07 D 403/12,
C 07 D 413/12

# (54) 2,4-Diamino-5-benzylpyrimidine, Verfahren zu ihrer Herstellung und diese enthaltende Arzneimittel.

(30) Priorität: **06.07.77 DE 2730467**

(43) Veröffentlichungstag der Anmeldung:
**24.01.79 Patentblatt 79/2**

(45) Bekanntmachung des Hinweises auf die Patenterteilung:
**12.08.81 Patentblatt 81/32**

(84) Benannte Vertragsstaaten:
**BE CH DE FR GB LU NL SE**

(56) Entgegenhaltungen:
**US - A - 2 649 449**
**US - A - 2 723 975**

**JOURNAL OF MEDICINAL AND PHARMACEUTICAL CHEMISTRY, vol. 5, Nov. 1962 Seiten 1.103—1.123 (The American Chemical Society): 5-Benzyl-2,4-diamino-pyrimidines as Antibacterial Agents. I. Synthesis and Antibacterial Activity in vitro: by B. Roth, E. A. Falco, G. H. Hitchings & S. R. M. Bushby**

(73) Patentinhaber: **BASF Aktiengesellschaft**
**Carl-Bosch-Strasse 38**
**D-6700 Ludwigshafen (DE)**

(72) Erfinder: **Gutsche, Klaus, Dr.**
**Ehmschenkamp 5**
**D-2084 Rellingen (DE)**
Erfinder: **Scharwaechter, Peter, Dr.**
**An der Duene 9**
**D-2082 Moorrege (DE)**
Erfinder: **Kohlmann, Wilhelm, Dr.**
**An der Duene 6**
**D-2082 Moorrege (DE)**
Erfinder: **Kroemer, Gerd, Dr.**
**verstorben, zuletzt wohnhaft Kaltenweide 100a**
**D-2200 Elmshorn (DE)**

**EP 0 000 334 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

2,4-Diamino-5-benzylpyrimidine, Verfahren zu ihrer Herstellung und diese enthaltende Arzneimittel

Die Erfindung betrifft 2,4-Diamino-5-benzylpyrimidine der allgemeinen Formel I

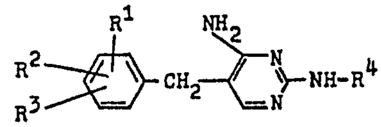

(I)

in der $R^1$, $R^2$ und $R^3$, die gleich oder verschieden voneinander sein können, Wasserstoff, Methyl, Methoxy oder Chlor bedeuten und $R^4$ einen Methylrest, der substituiert ist durch einen Alkoxyrest mit 1 bis 6 C-Atomen, dessen Alkylrest gegebenenfalls zusätzlich substituiert ist durch ein Chloratom oder eine Alkoxygruppe mit 1 bis 2 C-Atomen im Alkylrest, der wiederum durch einen Alkoxyrest mit 1 bis 4 C-Atomen substituiert sein kann, einen Allyloxyrest, einen Cyclohexyloxyrest oder einen Benzyloxyrest, bedeutet oder $R^4$ bedeutet Allyl oder einen Alkylrest mit 1 bis 3 C-Atomen, der durch Phenyl, Chlorphenyl, Hydroxy, Alkoxy mit 1 bis 2 C-Atomen, Dialkylamino mit 1 bis 2 C-Atomen im Alkyl oder einen Pyrrolidino- oder Morpholinorest substituiert ist, oder $R^4$ bedeutet einen 3-Alkylisoxazolyl-5-methyl-rest mit 1 bis 4 C-Atomen im Alkyl, sowie deren pharmakologisch verträglichen Säureadditionssalze mit hierfür üblichen Säuren.

Bevorzugt stehen die Substituenten $R^1$, $R^2$ und $R^3$ in der 3-, 4- und 5-Stellung des Benzolrings.

Ganz besonders bevorzugt sind dabei diejenigen Verbindungen der allgemeinen Formel I, in der $R^1$, $R^2$ und $R^3$ Methoxygruppen bedeuten.

Die Verbindungen der allgemeinen Formel I und deren Salze sind antimikrobiell wirksam bei durch Bakterien und Protozoen hervorgerufenen Krankheiten und potenzieren, kombiniert mit Sulfonamiden, deren antimikrobielle Wirkung. Sie können beispielsweise bei bakteriellen Erkrankungen der Atmungsorgane, Verdauungsorgane und Harnwege sowie bei Hals-, Nasen-, Ohreninfektionen und allgemein systemischen Infektionskrankheiten und bei Malaria verwendet werden.

Solche Sulfonamide sind beispielsweise:

2-Sulfanilamidopyrimidin, 2-Sulfanilamido-5-methoxypyrimidin, 4-Sulfanilamido-2,6-dimethoxypyrimidin, 3-Sulfanilamido-5-methylisoxazol, 2-Sulfanilamido-4,5-dimethyloxazol, 3-Sulfanilamido-6-methoxypyridazin, 4-Sulfanilamido-2,6-dimethylpyrimidin, 4-Sulfanilamido-5,6-dimethoxypyrimidin, 2-Sulfanilamido-3-methoxypyrazin.

Als übliche Säuren zur Bildung pharmakologisch verträglicher Salze kommen in Frage: Salzsäure, Schwefelsäure, Phosphorsäure, Salpetersäure, Essigsäure, Milchsäure, Weinsäure und Zitronensäure.

Bevorzugt werden jedoch die genannten anorganischen Säuren, insbesondere Salzsäure und Schwefelsäure, die mit den erfindungsgemäßen Substanzen besonders gut kristallisierende Salze bilden.

Die Verbindungen der allgemeinen Formel I und deren Salze können mit den beispielhaft genannten Sulfonamiden in verschiedenen Mischungsverhältnissen kombiniert werden, wobei das Verhältnis Substanz nach allgemeiner Formel I: Sulfonamid in dem Bereich 1:10 bis 5:1 variieren kann. Bevorzugte Mischungsverhältnisse sind jedoch 1:1 bis 1:5. Dabei kommen in der Regel als Dosierung 20 bis 500 mg eines Wirkstoffs der allgemeinen Formel I in Betracht.

Die Herstellung der erfindungsgemäßen Substanzen nach der allgemeinen Formel I geschieht in der Weise, daß man

a) eine Verbindung der allgemeinen Formel II

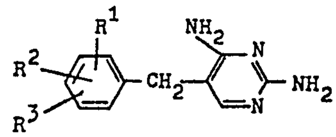

(II)

in der $R^1$, $R^2$ und $R^3$ die gleiche Bedeutung wie in der allgemeinen Formel I haben, mit einer Verbindung der allgemeinen Formel III

$$Hal—R^4 \qquad (III)$$

in der $R^4$ die gleiche Bedeutung wie in der allgemeinen Formel I hat, und Hal ein Halogenatom, insbesondere Cl oder Br bedeutet, umsetzt oder

b) eine Verbindung der allgemeinen Formel IV

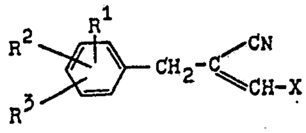

(IV)

in der $R^1$, $R^2$ und $R^3$ die gleiche Bedeutung wie in der allgemeinen Formel I haben und X eine Abgangsgruppe bedeutet, mit einer Verbindung der allgemeinen Formel V

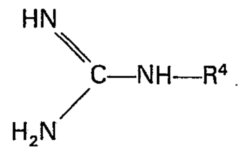

(V)

in der $R^4$ die gleiche Bedeutung wie in der allgemeinen Formel I hat, umsetzt oder

c) eine Verbindung der allgemeinen Formel VI

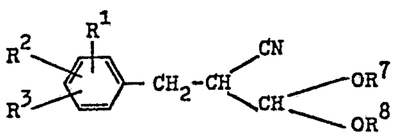

(VI)

in der $R^1$, $R^2$ und $R^3$ die gleiche Bedeutung wie in der allgemeinen Formel I haben und $R^7$ und $R^8$ niedere Alkylreste darstellen, mit einer Verbindung der allgemeinen Formel V umsetzt.

Gegenüber den zahlreichen literaturbekannten, über eine Ringschlußreaktion mit Verbindungen der allgemeinen Formel V verlaufenden Synthesemöglichkeiten sollen die Verfahren a) bis c) keine Einschränkungen darstellen.

Bei der Verfahrensweise a) wird im allgemeinen in einem aprotischen Verdünnungsmittel, wie beispielsweise Dioxan, Tetrahydrofuran, Benzol, Chlorbenzol, Chloroform oder Pyridin gearbeitet, wobei die Reaktionstemperaturen je nach Reaktivität der Verbindung der allgemeinen Formel III zwischen 0 und 200°C liegen.

Bei der Verfahrensweise b) wird in Alkoholen, vorzugsweise in Methanol oder Äthanol, oder in Dimethylformamid oder Dimethylsulfoxid als Lösungsmittel gearbeitet, wobei die Reaktionstemperaturen zwischen 50 und 150°C liegen. Temperaturen um 150°C sind dann erforderlich, wenn die Abgangsgruppe X eine schwer reagierend aliphatische Aminogruppe bedeutet. Die Abgangsgruppe in der allgemeinen Formel IV bedeutet eine Alkoxygruppe, vorzugsweise die Methoxy- und die Äthoxygruppe, oder eine sekundäre aliphatische Aminogruppe, vorzugsweise eine Morpholino- oder Dimethylaminogruppe, oder eine primäre aromatische Aminogruppe, vorzugsweise die Anilinogruppe oder den Imidazolyl-1-rest.

Zum Wirkungsnachweis wurden erfindungsgemäße Substanzen im Tierversuch am Modell der sogenannten Aronson-Sepsis, wobei mit Streptococcus agalactiae infiziert wird, geprüft und mit dem bekannten Trimethoprim verglichen. Hierzu wurden Gruppen von je 30 weiblichen Mäusen mit einer tödlichen Dosis von Streptococcus agalactiae 7941 infiziert und 2 Stunden nach der Infektion mit einer Mischung von 300 mg 2-Sulfanilamido-4,5-dimethyl-oxazol + 60 mg einer der erfindungsgemäßen Substanzen behandelt. Außer einer nicht behandelten Kontrollgruppe wurde eine zweite Gruppe mit dem als Referenzsubstanz dienenden Gemisch von 300 mg 2-Sulfanilamido-4,5-dimethyloxazol + 60 mg Trimethoprim behandelt. Nach 44 Stunden wurde die Zahl der überlebenden Tiere bestimmt und diese Zahl durch die Zahl der überlebenden aus der mit der Referenzsubstanz behandelten Gruppe dividiert. Der so erhaltene Zahlenwert (Trimethoprimfaktor) ist ein Maß für die Wirkung der erfindungsgemäßen Substanzen im Vergleich zum Trimethoprim. F = 2 bedeutet also, daß die Substanz doppelt so wirksam ist wie Trimethoprim. Aus der folgenden Tabelle geht eine Überlegenheit der erfindungsgemäßen Substanzen gegenüber dem Trimethoprim bis zum 3-fachen hervor.

Tabelle I

Allgemeine Formel

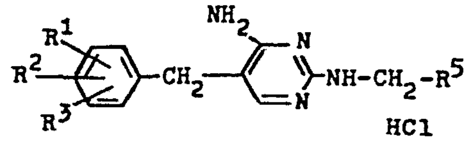

| Nr. | $R^1$ | $R^2$ | $R^3$ | $R^5$ | F |
|---|---|---|---|---|---|
| 1 | (3)—$OCH_3$ | (4)—$OCH_3$ | (5)—$OCH_3$ | —$OCH_3$ | 1,25 |
| 2 | (3)—$OCH_3$ | (4)—$OCH_3$ | (5)—$OCH_3$ | —O—$C_3H_7$(n) | 2.50 |
| 3 | (3)—$OCH_3$ | (4)—$OCH_3$ | (5)—$OCH_3$ | —O— 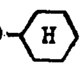 | 1,60 |
| 4 | (3)—$OCH_3$ | (4)—$OCH_3$ | (5)—$OCH_3$ | —O—$CH_2$ 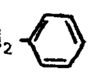 | 2,00 |
| 5 | (3)—$OCH_3$ | (4)—$OCH_3$ | (5)—$OCH_3$ | —O—$C_2H_4 \cdot Cl$ | 1,00 |
| 6 | (3)—$OCH_3$ | (4)—$OCH_3$ | (5)—$OCH_3$ | —$CH_2$ 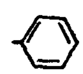 | 2,00 |
| 7 | (3)—$OCH_3$ | (4)—$OCH_3$ | (5)—$OCH_3$ | —O—$(CH_2)_5 \cdot CH_3$ | 1,10 |
| 8 | (3)—$OCH_3$ | (4)—$OCH_3$ | (5)—$OCH_3$ | —CH=$CH_2$ | 1,33 |
| 9 | (3)—$OCH_3$ | (4)—$OCH_3$ | (5)—$OCH_3$ | —O—$CH_2$—CH=$CH_2$ | 1,64 |
| 10 | (3)—$OCH_3$ | (4)—$OCH_3$ | (5)—$OCH_3$ | 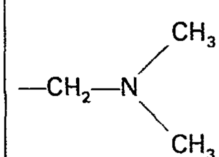 | 1,10 |
| 11 | (3)—$OCH_3$ | (4)—$OCH_3$ | (5)—$OCH_3$ | —$CH_2$— 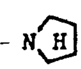 | 1,50 |
| 12 | (3)—$OCH_3$ | (4)—$OCH_3$ | (5)—$OCH_3$ | —$CH_2$— 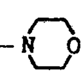 | 2,00 |
| 13 | (3)—$OCH_3$ | (4)—$OCH_3$ | (5)—$OCH_3$ | —$CH_2$—O—$C_2H_5$ | 3,00 |
| 14 | (3)—$OCH_3$ | (4)—$OCH_3$ | (5)—$OCH_3$ | —$CH_2$—$CH_2$—O—$C_2H_5$ | 1,00 |
| 15 | (3)—$OCH_3$ | (4)—$OCH_3$ | (5)—$OCH_3$ | 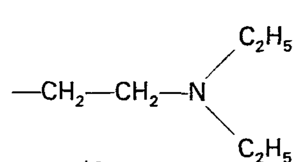 | 1,50 |
| 16 | (3)—$OCH_3$ | (4)—$OCH_3$ | (5)—$OCH_3$ | 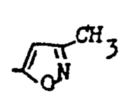 | 1,10 |
| 17 | H | (4)—$OCH_3$ | H | —O—$CH_2$—CH=$CH_2$ | 1,67 |
| 18 | (3)—$OCH_3$ | (4)—$OCH_3$ | H | —O—$CH_2$ 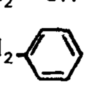 | 1,17 |
| 19 | 2—Cl | H | H | —O—$CH_2$—$CH_2$—$CH_2$—Cl | 1,50 |

Gegenstand der Erfindung sind demnach auch chemotherapeutische Mittel, die neben üblichen Träger- und Verdünnungsmitteln eine Verbindung der allgemeinen Formel I, insbesondere in Kombination mit einem Sulfonamid als Wirkstoffe enthalten, sowie die Verwendung der Verbindungen der allgemeinen Formel I als Sulfonamidpotentiatoren.

Die chemotherapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen.

Beispiel 1

2-Methoxymethylamino-4-amino-5-(3,4,5-trimethoxybenzyl)-pyrimidin-hydrochlorid

5,8 g 2,4-Diamino-5-(3,4,5-trimethoxybenzyl)-pyrimidin wurden in 60 ml of Pyridin bei 60°C gelöst und die Lösung bei dieser Temperatur tropfenweise mit 3,0 ml Chlordimethyläther versetzt. Anschließend wurde das Pyridin im Vakuum abdestilliert und der Rückstand aus 250 ml Äthanol umkristallisiert. Es wurden so 5,5 g (74% d.Th.) 2-Methoxymethylamino-4-amino-5-(3,4,5-trimethoxybenzyl)-pyrimidin-HCl mit dem Fp.: 227°C erhalten.

Beispiel 2

2-Cyclohexyloxymethylamino-4-amino-5-(3,4,5-trimethoxybenzyl)-pyrimidin-hydrochlorid

5,8 g 2,4-Diamino-5-(3,4,5-trimethoxybenzyl)-pyrimidin wurden in 100 ml Dioxan bei 80°C gelöst und die Lösung mit 2,97 g Chlormethylcyclohexyläther tropfenweise versetzt. Nach Beendigung der Zugabe wurde noch 30 Minuten bei 90°C gerührt. Der nach dem Abkühlen erhaltene Niederschlag wurde aus Methylglykol unter Zusatz von Äther umkristallisiert. Es wurden 6,8 g (77% d.Th.) 2-Cyclohexyloxymethylamino-4-amino-5-(3,4,5-trimethoxybenzyl)-pyrimidin-HCl mit dem Fp.: 208°C erhalten.

Analog Beispiel 1 und 2 wurden außerdem hergestellt:

3. 2 - Äthoxymethylamino - 4 - amino - 5 - (3,4,5 - trimethoxybenzyl) - pyrimidin - HCl mit dem Fp.: 206°C aus 2,4 - Diamino - 5 - (3,4,5, - trimethoxybenzyl) - pyrimidin und Chlormethyläthyläther.
4. 2 - n - Propyloxymethylamino - 4 - amino - 5 - (3,4,5 - trimethoxybenzyl) - pyrimidin - HCl mit dem Fp.: 249°C aus 2,4 - Diamino - 5 - (3,4,5 - trimethoxybenzyl) - pyrimidin und Chlormethyl - n - propyläther.
5. 2 - n - Butyloxymethylamino - 4 - amino - 5 - (3,4,5 - trimethoxy - benzyl) - pyrimidin - HCl mit dem Fp.: 235°C aus 2,4 - Diamino - 5 - (3,4,5 - trimethoxybenzyl) - pyrimidin und Chlormethyln - butyläther.
6. 2 - n - Hexyloxymethylamino - 4 - amino - 5 - (3,4,5 - trimethoxybenzyl) - pyrimidin - HCl mit dem Fp.: 228°C aus 2,4 - Diamino - 5 - (3,4,5 - trimethoxybenzyl) - pyrimidin und Chlormethyl - n - hexyläther.
7. 2 - Allyloxymethylamino - 4 - amino - 5 - (3,4,5 - trimethoxybenzyl) - pyrimidin - HCl mit dem Fp.: 220 bis 222°C aus 2,4 - Diamino - 5 - (3,4,5 - trimethoxybenzyl) - pyrimidin und Chlormethylallyläther.
8. 2 - (β - Chloräthoxymethylamino) - 4 - amino - 5 - (3,4,5 - trimethoxybenzyl) - pyrimidin - HCl mit dem Fp.: 218°C aus 2,4 - Diamino - 5 - (3,4,5 - trimethoxybenzyl) - pyrimidin und Chlormethyl - β - chloräthyläther.
9. 2 - (2 - Chlor - 1 - methyl - äthoxymethylamino) - 4 - amino - 5 - (3,4,5 - trimethoxybenzyl) - pyrimidin - HCl mit dem Fp.: 230°C aus 2,4 - Diamino - 5 - (3,4,5 - trimethoxybenzyl) - pyrimidin und Chlormethyl - (2 - chlor - 1 - methyläthyl) - äther.
10. 2 - Benzyloxymethylamino - 4 - amino - 5 - (3,4,5 - trimethoxybenzyl) - pyrimidin - HCl mit dem Fp.: 227°C aus 2,4 - Diamino - 5 - (3,4,5, - trimethoxybenzyl) - pyrimidin und Chlormethylbenzyläther.
11. 2 - (β - Methoxyäthoxymethylamino) - 4 - amino - 5 - (3,4,5 - trimethoxybenzyl) - pyrimidin - HCl mit dem Fp.: 226°C aus 2,4 - Diamino - 5 - (3,4,5 - trimethoxybenzyl) - pyrimidin und Chlormethyl - β - methoxyäthyläther.
12. 2 - (β - Äthoxyäthoxymethylamino) - 4 - amino - 5 - (3,4,5 - trimethoxybenzyl) - pyrimidin - HCl mit dem Fp.: 216°C aus 2,4 - Diamino - 5 - (3,4,5 - trimethoxybenzyl) - pyrimidin und Chlormethyl - β -äthoxyäthyläther.
13. 2 - Cyclohexyloxymethylamino - 4 - amino - 5 - (4 - methoxybenzyl) - pyrimidin - HCl mit dem Fp.: 297°C aus 2,4 - Diamino - 5 - (4 - methoxy) - pyrimidin und Chlormethylcyclohexyläther.
14. 2 - Benzyloxymethylamino - 4 - amino - 5 - (3,4, - dimethoxybenzyl) - pyrimidin - HCl mit dem Fp.: 182°C aus 2,4 - Diamino - 5 - (3,4 - Dimethoxybenzyl) - pyrimidin und Chlormethylbenzyläther.

15. 2 - (β - Chloräthoxymethylamino) - 4 - amino - 5 - (2 - chlorbenzyl) - pyrimidin - HCl mit dem Fp.: 222°C aus 2,4 - Diamino - 5 - (2 - chlorbenzyl) - pyrimidin und Chlormethyl - β - chloräthyläther.
16. 2 - (β - Äthoxyäthoxymethylamino) - 4 - amino - 5 - (4 - chlorbenzyl) - pyrimidin - HCl mit dem Fp.: 218°C aus 2,4 - Diamino - 5 - (4 - chlorbenzyl) - pyrimidin und Chlormethyl - β- äthoxyäthyläther.
17. 2 - Allyloxymethylamino - 4 - amino - 5 - (2,4 - dimethoxybenzyl) - pyrimidin - HCl mit dem Fp.: 200°C aus 2,4 - Diamino - 5 - (2,4 - dimethoxybenzyl) - pyrimidin und Chlormethylallyläther.
18. 2 - (3 - Methylisoxazolyl - 5) - methylamino - 4 - amino - 5 - (3,4,5 - trimethoxybenzyl) - pyrimidin - HCl mit dem Fp.: 290°C aus 2,4 - Diamino - 5 - (3,4,5 - trimethoxybenzyl) - pyrimidin und 3 - Methyl - 5 - chlormethyl - isoxazol.
19. 2 - (3 - Äthylisoxazolyl - 5) - methylamino - 4 - amino - 5 - (3,4,5 - trimethoxybenzyl) - pyrimidin - HCl mit dem Fp.: 291°C aus 2,4 - Diamino - 5 - (3,4,5 - trimethoxybenzyl) - pyrimidin und 3 - Äthyl - 5 - chlormethyl - isoxazol.
20. 2 - (3 - Isopropylisoxazolyl - 5) - methylamino - 4 - amino - 5 - (3,4,5 - trimethoxybenzyl) - pyrimidin - HCl mit dem Fp.: 290°C aus 2,4 - Diamino - 5 - (3,4,5 - trimethoxybenzyl) - pyrimidin und 3 - Isopropyl - 5 - chlormethyl - isoxazol.
21. 2 - (3 - Tertiärbutylisoxazolyl - 5) - methylamino - 4 - amino - 5 - (3,4,5 - trimethoxybenzyl) - pyrimidin - HCl mit dem Fp.: 280°C aus 2,4 - Diamino - 5 - (3,4,5 - trimethoxybenzyl) - pyrimidin und 3 - Tertiärbutyl - 5 - chlormethyl - isoxazol.

Beispiel 22

2-Benzylamino-4-amino-5-(3,4,5-trimethoxybenzyl)-pyrimidin

5 g α-Anilino-β-(3,4,5-trimethoxybenzyl)-acrylnitril, 5,9 g Benzylguanidiniumsulfat und 1,6 g Natriummethylat wurden in 50 ml Äthanol 4 Stunden unter Rückfluß zum Sieden erhitzt. Danach wurde das Gemisch mit 10 ml Wasser versetzt und nach dem Abkühlen die Kristalle abgesaugt und mit Wasser gewaschen.

Nach Umkristallisieren des Produktes aus Isopropanol wurden 4,1 g (72% d.Th.) 2-Benzylamino-4-amino-5-(3,4,5-trimethoxybenzyl)-pyrimidin mit dem Fp.: 135°C erhalten.

Analog Beispiel 22 wurden erhalten:

23. 2 - Allylamino - 4 - amino - 5 - (3,4,5 - trimethoxybenzyl) - pyrimidin mit dem Fp.: 132°C unter Verwendung von Allylguanidinsulfat.
24. 2 - (Phenäthyl - β - amino) - 4 - amino - 5 - (3,4,5 - trimethoxybenzyl) - pyrimidin mit dem Fp.: 124°C unter Verwendung von Phenäthylguanidinsulfat.
25. 2 - (4 - Chlorbenzylamino) - 4 - amino - 5 - (3,4,5 - trimethoxybenzyl) - pyrimidin mit dem Fp.: 143°C unter Verwendung von 4-Chlorbenzylguanidinsulfat.
26. 2 - (β - Dimethylaminoäthylamino) - 4 - amino - 5 - (3,4,5 - trimethoxybenzyl) - pyrimidin mit dem Fp.: 139°C unter Verwendung von β - Dimethylaminoäthylguanidinsulfat.
27. 2 - (β - Morpholinoäthylamino) - 4 - amino - 5 - (3,4,5 - trimethoxybenzyl) - pyrimidin mit dem Fp.: 140°C unter Verwendung von β - Morpholinäthylguanidinsulfat.
28. 2 - (β - Pyrrolidinoäthylamino) - 4 - amino - 5 - (3,4,5 - trimethoxybenzyl) - pyrimidin mit dem Fp.: 130°C unter Verwendung von β - Pyrrolidinoäthylguanidinsulfat.
29. 2 - (3 - Dimethylamino - n - propyl - 1 - amino) - 4 - amino - 5 - (3,4,5 - trimethoxybenzyl) - pyrimidin mit dem Fp.: 139°C unter Verwendung von 3 - Dimethylamino - n - propyl - 1 - guanidinsulfat.
30. 6,4 g α - Cyano - β - (3,4,5 - trimethoxyphenyl) - propionaldehyddimethylacetal, 3 g β - Hydroxyäthylguanidinsulfat und 1,1 g Natriummethylat wurden in 100 ml Äthanol 5 Stunden unter Rückfluß zum Sieden erhitzt. Danach wurde das Äthanol abdestilliert und der Rückstand in 100 ml Wasser gelöst. Durch Extraktion der Lösung mit Chloroform wurden 3,4 g (50% d.Th.) 2 - (β - Hydroxyäthylamino) - 4 - amino - 5 - (3,4,5 - trimethoxybenzyl) - pyrimidin mit dem Fp.: 146°C nach Umkristallisation aus Isopropanol erhalten.
31. 5,6 g α - (3,4,5 - Trimethoxybenzyl) - β - dimethylaminoacrylnitril, 3,8 g β - Äthoxyäthylguanidinsulfat und 2 g Natriummethylat wurden in 100 ml Dimethylsulfoxid 3 Stunden bei 150°C gerührt. Danach wurde das Dimethylsulfoxid im Vakuum abdestilliert und der Rückstand mit 100 ml Wasser versetzt. Das ölige Produkt wurde mit Chloroform extrahiert und der Rückstand nach dem Einengen der Extrakte mehrfach aus Essigester Isopropyläther umkristallisiert. Man erhielt so 1,4 g (20% d.Th.) 2-(β-Äthoxyäthylamino)-4-amino-5-(3,4,5-trimethoxybenzyl)-pyrimidin mit dem Fp.: 147°C.
32. 12 g β-Imidazolyl - 1 - propionitril, 6 g Natriummethylat und 19,6 g 3,4,5 - Trimethoxybenzaldehyd wurden 12 Stunden in 200 ml Methanol unter Rückfluß zum Sieden erhitzt. Danach wurden 37 g (3 - Äthoxy - n - propyl - 1) - guanidinsulfat und weitere 6 g Natriummethylat zugegeben, das Methanol langsam abdestilliert und der Rückstand 2 Stunden bei 110°C gerührt.

Das Reaktionsgemisch wurde mit 200 ml Wasser verrührt und das halbfeste Produkt mit Chloroform extrahiert. Der Rückstand vom Chloroformextrakt wurde aus Essigester/Isopropyläther umkristallisiert. Man erhielt so 12 g (32% d.Th.) 2 - (3 - Äthoxy - n - propyl - 1 - amino) - 4 - amino - 5 - (3,4,5 - trimethoxybenzyl) - pyrimidin mit dem Fp.: 118°C.

33. Analog Beispiel 1 wurde erhalten:
2 - [β(β - Methoxyäthoxy) - äthoxymethylamino] - 4 - amino - 5 - (3,4,5 - trimethoxybenzyl) - pyrimidin - hydrochlorid mit dem Fp. 206 bis 209°C aus Trimethoprim und β - (β - Methoxyäthoxy) - äthylchlormethyläther
($R^4 = -CH_2-O-C_2H_4-O-C_2H_4-OCH_3$).

34. Analog Beispiel 1 wurde erhalten:
2 - [β(β - n - Butoxyäthoxy) - äthoxymethylamino] - 4 - amino - 5 - (3,4,5 - trimethoxy) - benzylpyrimidin - hydrochlorid mit dem Fp. 213°C aus Trimethoprim und β - (β - n - Butoxyäthoxy) - äthylchlormethyläther
($R^4 = -CH_2-O-C_2H_4-O-C_2H_4-O-C_4H_9(n)$).

Beispiele für Zubereitungen

1. 400 mg 2-Sulfanilamido-4,5-dimethyloxazol
80 mg 2-Benzyloxymethylamino-4-amino-5-(3,4,5-trimethoxybenzyl)-pyrimidin
20 mg Maisstärke
10 mg Gelatine
8 mg Talkum
2 mg Magnesiumstearat
20 mg Primojel

Die Wirkstoffe werden mit Maisstärke gemischt und mit wäßriger Gelatinelösung granuliert. Das trockene Granulat wird gesiebt und mit den Zuschlägen vermischt. Aus dieser Mischung werden in üblicher Weise Tabletten gepreßt.

2. 160 mg 2-Sulfanilamido-5-methoxy-pyrimidin
80 mg 2-n-Hexyloxymethylamino-4-amino-5-(3,4,5-trimethoxybenzyl)-pyrimidin
5 mg Gelatine
30 mg Maisstärke
4 mg Talkum
1 mg Magnesiumstearat

Die Wirkstoffe werden mit wäßriger Gelatinelösung granuliert und nach dem Trocknen mit Maisstärke, Talkum und Magnesiumstearat vermischt. Aus dieser Mischung werden in üblicher Weise Tabletten gepreßt.

3. 4,00 g 2-Sulfanilamido-5-methoxy-pyrimidin
2,00 g 2-n-Hexyloxymethylamino-4-amino-5-(3,4,5-trimethoxybenzyl)-pyrimidin
1,9 g Tylose® C 30
30,0 g Zucker
10,0 g Glycerin
2,5 g Bentonit
0,06 g Aroma
0,04 g Nipagin ®M
0,06 g Nipasol ®Natrium
ad 100,00 g demineralisiertes Wasser

Die feinst gemahlenen Wirkstoffe werden in dem wäßrigen Tylose-Schleim suspendiert. Anschließend werden alle anderen Bestandteile unter Rühren nacheinander zugegeben. Zum Schluß wird mit Wasser auf 100,0 g aufgefüllt.

**Patentansprüche**

1. 2,4-Diamino-5-benzylpyrimidine der allgemeinen Formel I

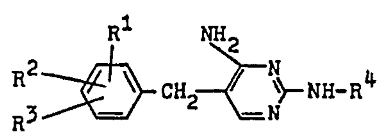

(I)

in der $R^1$, $R^2$ und $R^3$, die gleich oder verschieden voneinander sein können, Wasserstoff, Methyl, Methoxy oder Chlor bedeuten und $R^4$ einen Methylrest, der substituiert ist durch einen Alkoxyrest mit 1 bis 6 C-Atomen, dessen Alkylrest gegebenenfalls zusätzlich substituiert ist durch ein Chloratom oder eine Alkoxygruppe mit 1 bis 2 C-Atomen im Alkylrest, der wiederum durch einen Alkoxyrest mit 1 bis 4 C-Atomen substituiert sein kann, einen Allyloxyrest, einen Cyclohexyloxyrest oder einen Benzyloxyrest, bedeutet oder $R^4$ bedeutet Allyl oder einen Alkylrest mit 1 bis 3 C-Atomen, der durch Phenyl, Chlorphenyl, Hydroxy, Alkoxy mit 1 bis 2 C-Atomen, Dialkylamino mit 1 bis 2 C-Atomen im Alkyl oder einen Pyrrolidino-oder Morpholinorest substituiert ist, oder $R^4$ bedeutet einen 3-Alkylisoxazolyl-5-methyl-rest mit 1 bis 4 C-Atomen im Alkyl, sowie deren pharmakologisch verträglichen Säureadditionssalze mit hierfür üblichen Säuren.

2. Verfahren zur Herstellung von Verbindungen gemäß Anspruch 1, dadurch gekennzeichnet, daß man

a) eine Verbindung der allgemeinen Formel II

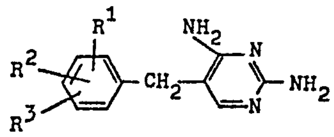

(II)

in der $R^1$, $R^2$ und $R^3$ die gleiche Bedeutung wie in Formel I haben, mit einer Verbindung der allgemeinen Formel III umsetzt

$$Hal—R^4 \qquad (III)$$

in der $R^4$ die gleiche Bedeutung wie in der allgemeinen Formel I hat, und Hal ein Halogenatom bedeutet, oder

b) eine Verbindung der allgemeinen Formel IV

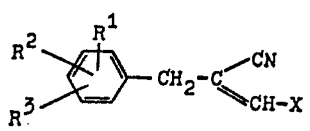

(IV)

in der $R^1$, $R^2$ und $R^3$ die gleiche Bedeutung wie in der allgemeinen Formel I haben und X eine Alkoxygruppe, eine aliphatische sekundäre Aminogruppe, die Anilinogruppe oder einen Imidazolyl-1-Rest bedeutet, mit einer Verbindung der allgemeinen Formel V umsetzt

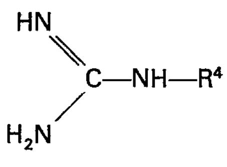

(V)

in der $R^4$ die gleiche Bedeutung wie in der allgemeinen Formel I hat,

oder

c) eine Verbindung der allgemeinen Formel VI

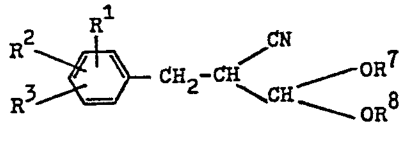

(VI)

in der $R^1$, $R^2$ und $R^3$ die gleiche Bedeutung wie in der allgemeinen Formel I haben, $R^7$ und $R^8$ niedere Alkylreste darstellen, mit einer Verbindung der allgemeinen Formel V umsetzt und die erhaltene Verbindung der allgemeinen Formel I, falls erwünscht, in ein pharmakologisch verträgliches Säureadditionssalz umwandelt.

3. Arzneimittel, enthaltend eine Verbindung gemäß Anspruch 1, gegebenenfalls zusammen mit einem Sulfonamid, und nichttoxischen, therapeutisch verträglichen festen oder flüssigen Trägerstoffen und galenischen Hilfstoffen.

**Claims**

1. 2,4-Diamino-5-benzylpyrimidines of the general formula I

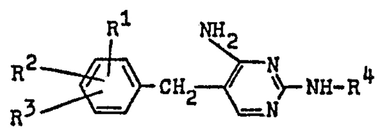

(I)

where $R^1$, $R^2$ and $R^3$, which may be identical or different, are hydrogen, methyl, methoxy or chlorine, and $R^4$ is methyl which is substituted by alkoxy of 1 to 6 carbon atoms, whereof the alkyl may additionally be substituted by a chlorine atom or an alkoxy group of 1 or 2 carbon atoms which in turn may be substituted by alkoxy of 1 to 4 carbon atoms, or by allyloxy, cyclohexoxy or benzyloxy, or $R^4$ is allyl or alkyl of 1 to 3 carbon atoms which is substituted by phenyl, chlorophenyl, hydroxyl, alkoxy of 1 or 2 carbon atoms, dialkylamino (where alkyl is of 1 or 2 carbon atoms) or pyrrolidino or morpholino, or $R^4$ is 3-alkylisoxazolyl-5-methyl, where alkyl is of 1 to 4 carbon atoms, and their pharmacologically acceptable addition salts with acids conventionally used for this purpose.

2. A process for the manufacture of a compound as claimed in claim 1, characterized in that

a) a compound of the general formula II

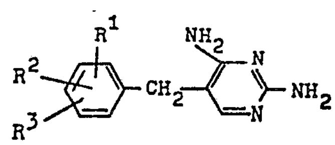

(II)

where $R^1$, $R^2$ and $R^3$ have the same meanings as in formula I, is reacted with a compound of the general formula III

$$\mathrm{Hal{-}R^4} \quad \text{(III)}$$

where $R^4$ has the same meaning as in formula I, and Hal is halogen, or

b) a compound of the general formula IV

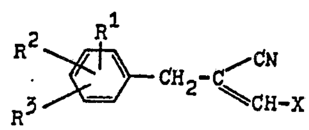

(IV)

where $R^1$, $R^2$ and $R^3$ have the same meanings as in formula I and X is alkoxy, aliphatic secondary amino, anilino or imidazol-1-yl, is reacted with a compound of the general formula V

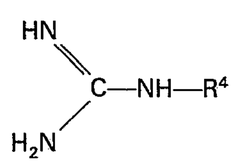

(V)

where $R^4$ has the same meaning as in formula I, or

c) a compound of the general formula VI

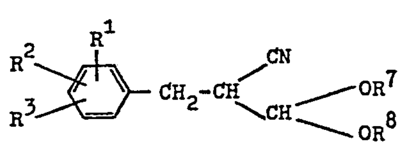

(VI)

where $R^1$, $R^2$ and $R^3$ have the same meanings as in formula I and $R^7$ and $R^8$ are lower alkyl, is reacted

with a compound of the general formula V, and the resulting compound of the general formula I may or may not be converted into a pharmacologically acceptable addition salt with an acid.

3. A drug containing a compound as claimed in claim 1, with or without a sulfonamide and nontoxic, therapeutically acceptable solid or liquid carriers and galenical assistants.

**Revendications**

1. 2,4-diamino-5-benzylpyrimidines de formule générale I

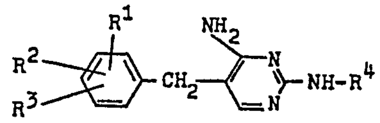

(I)

dans laquelle $R^1$, $R^2$ et $R^3$ peuvent être identiques ou différents les uns des autres et représentent hydrogène, méthyle, méthoxy ou chlore et $R^4$ représente un reste méthyle qui est substitué par un reste alcoxy en $C_1$ à $C_6$, dont le reste alkyle est en outre éventuellement substitué par un atome de chlore ou un groupe alcoxy ayant 1 ou 2 atomes C dans le reste alkyle, qui à son tour peut être substitué par un reste alcoxy ayant 1 à 4 atomes de carbone, un reste allyloxy, un reste cyclohexyloxy ou un reste benzyloxy, ou bien $R^4$ représente allyle ou un reste alkyle en $C_1$ à $C_3$ qui est substitué par phényle, chlorphényle, hydroxy, alcoxy en $C_1$ à $C_2$, dialkylamino en $C_1$ à $C_2$ dans l'alkyle ou un reste pyrrolidino ou morpholino, ou bien $R^4$ représente un reste 3-alkylisoxazolyl-5-méthyle ayant 1 à 4 atomes de C dans l'alkyle, ainsi que les sels d'addition d'acide compatibles du point de vue pharmacologique, avec des acides usuels pour cela.

2. Procédé pour la préparation de composés selon la revendication 1, caractérisé par le fait que:

a) on fait réagir un composé de formule générale II

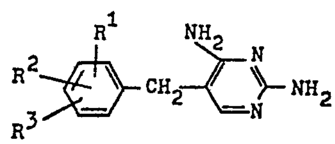

(II)

dans laquelle $R^1$, $R^2$, $R^3$ ont la même signification que dans la formule I, avec un composé de formule générale III

$$Hal—R^4 \qquad (III)$$

dans laquelle $R^4$ a la même signification que dans la formule I est Hal est un atome d'halogène,

ou bien

b) on fait réagir un composé de formule générale IV

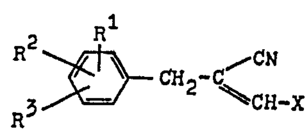

(IV)

dans laquelle $R^1$, $R^2$ et $R^3$ ont la même signification que dans la formule I et X est un groupe alcoxy, un groupe amino secondaire aliphatique, le groupe anilino ou un reste imidazolyl-1, avec un composé de formule générale V

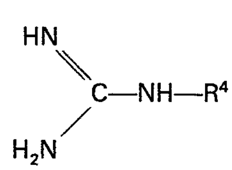

(V)

dans laquelle $R^4$ a la même signification que dans la formule I,

ou bien

c) on fait réagir un composé de formule générale VI

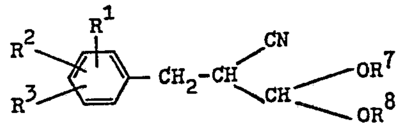

(VI)

dans laquelle $R^1$, $R^2$ et $R^3$ ont la même signification que dans la formule I, $R^7$ et $R^8$ représentent des restes alkyle inférieur, avec un composé de formule générale V et le composé obtenu, de formule générale I, est transformé, si on le désire, en un sel d'addition d'acide compatible du point de vue pharmacologique.

3. Médicament contenant un composé selon la revendication 1, éventuellement avec un sulfonamide et des supports non toxiques solides ou liquides et compatibles du point de vue thérapeutique et avec des adjuvants galéniques.